# EUROPEAN PATENT SPECIFICATION

(11) **EP 2 488 179 B1**
(45) Date of publication and mention of the grant of the patent: **18.02.2015**
(21) Application number: 10823621.7
(22) Date of filing: 15.10.2010
(51) Int. Cl.: A61K 31/4462, A61K 31/704, A61K 45/06

(54) **NRF2 INHIBITORS AND USE THEREOF**
NRF2-HEMMER UND IHRE VERWENDUNG
INHIBITEURS DE NRF2 ET UTILISATIONS DE CEUX-CI

(30) Priority: 15.10.2009 KR 20090098417
(43) Date of publication of application: 22.08.2012
(73) Proprietor: Korea Institute of Radiological & Medical Sciences, Seoul 139-709 (KR)
(72) Inventor: SONG, Jie Young, Seoul 122-737 (KR); YUN, Yeon Sook, Seoul 135-837 (KR); AHN, Ji Yeon, Seoul 136-777 (KR); JUNG, In Sung, Seoul 131-781 (KR); LEE, Sae Lo Oom, Seoul 140-869 (KR); PARK, Sarah, Seoul 135-776 (KR); LIM, Min Jin, Seoul 139-240 (KR); KIM, Mi Hyoung, Seoul 139-240 (KR)
(74) Representative: Potter Clarkson LLP
(86) International application number: PCT/KR2010/007072
(87) International publication number: WO 2011/046382

(56) References cited:
- WO-A2-2008/012534
- US-A- 3 634 437
- US-A- 6 120 994
- CULLINAN, S.B. ET AL.: 'Nrf2 is a direct PERK substrate and effector of PERK-dependent cell survival' MOLECULAR AND CELLULAR BIOLOGY vol. 23, no. 20, 2003, pages 7198 - 7209, XP008156392
- RUSHMORE, T. H. ET AL.: 'The antioxidant responsive element' JOURNAL OF BIOLOGICAL CHEMISTRY vol. 266, no. 18, 1991, pages 11632 - 11639, XP008156394

<110> Korea Institute of **Radiological & Medical** Sciences
<120> Nrf2 inhibitors and use thereof
<130> LEEBC/P51227EP
<140> EP10823621.7
   <141> 2010-10-15
<150> KR10-2009-0098417
   <151> 2009-10-15
<160> 12
<170> KopatentIn 1.71
<210> 1
   <211> 41
   <212> DNA
   <213> Artificial Sequence
<220>
   <223> 1xARE
<400> 1
   gggggggtac cctgtgctga gtcactggag acgcgtgggg g 41
<210> 2
   <211> 41
   <212> DNA
   <213> Artificial Sequence
<220>
   <223> 1xARE
<400> 2
   cccccacgcg tctccagtga ctcagcacag ggtacccccc c 41
<210> 3
   <211> 60
   <212> DNA
   <213> Artificial Sequence
<220>
   <223> 2xARE
<400> 3
<210> 4
   <211> 60
   <212> DNA
   <213> Artificial Sequence
<220>
   <223> 2xARE
<400> 4
<210> 5
   <211> 98
   <212> DNA
   <213> Artificial Sequence
<220>
   <223> 4xARE
<400> 5
   gggggggtac cctgtgctga gtcactggag ctgtgctgag tcactggagc tgtgctgagt 60
   cactggagct gtgctgagtc actggagacg cgtggggg 98
<210> 6
   <211> 98
   <212> DNA
   <213> Artificial Sequence
<220>
   <223> 4xARE
<400> 6
   cccccacgcg tctccagtga ctcagcacag ctccagtgac tcagcacagc tccagtgact 60
   cagcacagct ccagtgactc agcacagggt accccccc 98
<210> 7
   <211> 50
   <212> DNA
   <213> Artificial Sequence
<220>
   <223> 2(1/2)xARE
<400> 7
   gggggggtac cgtgctgagt cactggtgct gagtcactga cgcgtggggg 50
<210> 8
   <211> 50
   <212> DNA
   <213> Artificial Sequence
<220>
   <223> 2(1/2)xARE
<400> 8
   cccccacgcg tcagtgactc agcaccagtg actcagcacg gtaccccccc 50
<210> 9
   <211> 78
   <212> DNA
   <213> Artificial Sequence
<220>
   <223> 4(1/2)xARE
<400> 9
   gggggggtac cgtgctgagt cactggtgct gagtcactgg tgctgagtca ctggtgctga 60
   gtcactgacg cgtggggg 78
<210> 10
   <211> 78
   <212> DNA
   <213> Artificial Sequence
<220>
   <223> 4(1/2)xARE
<400> 10
   cccccacgcg tcagtgactc agcaccagtg actcagcacc agtgactcag caccagtgac 60
   tcagcacggt accccccc 78
<210> 11
   <211> 24
   <212> DNA
   <213> Artificial Sequence
<220>
   <223> forward primer for HO-1
<400> 11
   aagattgccc agaaagccct ggac 24
<210> 12
   <211> 24
   <212> DNA
   <213> Artificial Sequence
<220>
   <223> reverse primer for HO-1
<400> 12
   aactgtcgcc accagaaagc tgag 24

## Description

### Technical Field

The present invention relates to a composition for aiding an anti-cancer agent and radiation therapy comprising a nuclear factor E2 factor related-factor 2 (Nrf2) inhibitor.

The present invention is derived from a research project supported by the radiation technology development fund of the Ministry of Education, Science, and Technology [Project ID: M2070202000108N020200110 and 20090062246, Title: Development of combination therapy with radiation and effectiveness evaluation technoloy)].

### Background Art

Currently, cancer tops the list of mortality and has a cure rate of approximately 50% by palliative treatments such as surgery, radiation therapy, and chemotherapy. About 35% of all cancer patients in Korea and about half of all cancer patients in USA receive radiation therapy, and the number of cancer patients receiving the radiation therapy increase every year. The radiation therapy becomes more important in cancer treatment. However, during the treatment, cancer cells become resistant against anti-cancer agents or radiation, or high dose of radiation cause damages to normal tissues. Thus, research into an enhancer or adjuvant that increases therapeutic effect of anti-cancer agents or radiation therapy and minimizes development of resistance against anti-cancer treatment and influence on normal tissues has been intensively pursued.

Increase in activity of a nuclear factor E2 factor-related factor 2 (Nrf2) transcription factor caused by electrophile of an anti-cancer agent and an oxygen free radical signal by radiation may inhibit cancer treatment by anti-cancer agents and radiation. In particular, in lung cancer cells, the Nrf2 transcription factor is continuously activated so that therapeutic effect may not work (Singh A, Misra V, Thimmulappa RK, Lee H, Ames S, Hoque MO, Herman JG, Baylin SB, Sidransky D, Gabrielson E, Brock MV, Biswal S. Dysfunctional KEAP1-NRF2 interaction in non-small-cell lung cancer. PLoS Med. 2006 Oct;3(10):e420).

Doxorubicin (Adriamycin) is an anti-cancer agent that acts as topoisomerase II by inducing the generation of active oxygen species (ROS) and damaging DNA of cancer cells. Even though Doxorubicin is very effective for treatment of solid cancers such as lung cancer and colorectal cancer, and blood cancer such as leukemia, many cancer cells having high activity of the Nrf2 transcription factor show resistance against Dox-orubicin since the expression of an antioxidant enzyme such as heme oxygenase-1 (HO-1) that offsets the mechanism of Doxorubicin is induced. Thus, if the activity of Nrf2 is effectively inhibited, the resistance of cancer cells against Doxorubicin may be reduced to improve anti-cancer effect (Wang XJ, Sun Z, Villeneuve NF, Zhang S, Zhao F, Li Y, Chen W, Yi X, Zheng W, Wondrak GT, Wong PK, Zhang DD, Nrf2 enhances resistance of cancer cells to chemotherapeutic drugs, the dark side of Nrf2, Carcinogenesis. 2008 Jun;29(6): 1235-43).

Even though cancer may be cured by radiation therapy that induces the generation of active oxygen species, cancer cells secrete a large amount of antioxidant enzymes that remove the active oxygen species and thus may develop resistance against the radiation therapy. The expression of most of the antioxidant enzymes in the cancer cells is regulated by the Nrf2 transcription factor. If there is no oxidative stress, cytoplasmic Nrf2 binds to Keap1 protein to be degraded by continuous ubiquitination, so that the activity of the Nrf2 may be inhibited. On the other hand, if there is excess oxidative stress, the Nrf2 is separated from the Keap1 protein and translocated into nucleus. In the nucleus, the Nrf2 binds to antioxidant response element (ARE) present in an antioxidant enzyme gene promoter, leading to expression of antioxidant enzymes to remove the oxidative stress. However, in cancer cells, particularly, in lung cancer and prostate cancer cells, the Nrf2 is translocated into a nucleus and actively leads to overexpression of antioxidant enzymes even when there is no oxidative stress, due to mutation of Keap1 protein. Since the activity of the Nrf2 is further promoted by treatment with the anti-cancer agent and radiation therapy, activation of the Nrf2 has become a main cause behind the resistance against the treatment with anti-cancer agents and radiation therapy.

WO 2008012534 discloses an assay to identify compounds which may be of use in conjuction with cancer chemotherapeutic agents and/or antiproliferative agents. There is also disclosed a class of compounds identified by the assay which may be used in combination therapy, with current and novel agents, to treat cancers and other diseases associated with abnormal host cell proliferation, such as psoriasis.

To find inhibitor of the Nrf2 transcription factors, we have developed a luciferase vector system including an ARE binding site for screening an Nrf2 inhibitor.

### Disclosure of Invention

### Technical Problem

The present invention provides a composition for use in aiding an anti-cancer agent or radiation therapy comprising a nuclear factor E2 factor related-factor 2 (Nrf2) inhibitor which is 3-[(4-biphenyloxy)methyl]piperidine, by incrreasing sensitivity of cancer cells to the anti-cancer agent or radiation therapy when the composition is used in combination with the anti-cancer agent or radiation therapy.

The present disclosure also provides a method of screening an Nrf2 inhibitor.

### Solution to Problem

According to an aspect of the present invention, there is provided a composition for aiding an anti-cancer agent comprising the nuclear factor E2 factor related-factor 2 (Nrf2) inhibitor, which is 3-[(4-biphenylyloxymethyl]piperidine represented by Formula I below or a pharmaceutically acceptable salt thereof.

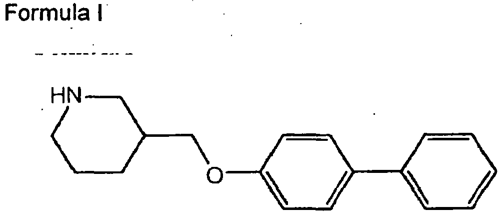

According to another aspect of the present invention, there is provided a composition for aiding radiation therapy comprising the Nrf2 inhibitor 3-[(4-biphenylyloxy)methyl]piperidine represented by Formula I below or a pharmaceutically acceptable salt thereof. 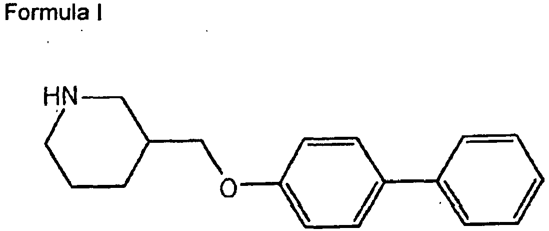 The Nrf2 plays an important role in transcriptional activation of antioxidant enzymes related genes regulated by antioxidant response element (ARE) site where Nrf2 binds, and the genes regulated by the ARE site for antioxidant enzymes. In general, anti-cancer agent or radiation therapy leads to apoptosis of cancer cells by inducing the generation of reactive oxygen species in cancer cells. The expression level of antioxidant enzymes in cancer cells is higher than that in normal cells, and activity of the Nrf2 is increased by anti-cancer therapy. Thus, the action of active oxygen species in cancer cells is inhibited by the antioxidant enzymes, reducing therapeutic effect of anti-cancer therapy. Given this, if the anti-cancer therapy is employed in combination with the Nrf2 inhibitor, the therapeutic effect may be improved.

The term "composition for aiding an anti-cancer agent" used herein refers to a composition that improves therapeutic effect of an anti-cancer agent by increasing sensitivity of cancer cells to the anti-cancer agent when the composition is used in combination with the anti-cancer agent.

In an embodiment of the present invention, the anti-cancer agent may be selected from the group consisting of cysplatin, doxorubicin, vinblastine, fluorouracil, irinotecan, paclitaxel, docetaxel, vinorelbine, and gemcitabine.

The term "composition for aiding radiation therapy" used herein refers to a composition that improves therapeutic effect of radiation therapy by increasing sensitivity of cancer cells to radiation when the composition is used in combination with the radiation therapy.

In an embodiment of the present invention, it was found that when the composition comprising 3-[(4-biphenylyloxy)methyl]piperidine or a pharmaceutically acceptable salt thereof was applied to cancer cell lines such as lung cancer, colorectal cancer, brain cancer, uterine cancer, and breast cancer cell lines and then treatment with the anti-cancer agent or radiation therapy was conducted, the death of the cancer cells was synergistically increased.

In an embodiment of the present invention, 3-[(4-biphenylyloxy)methyl]piperidine may be used alone, or may be used as pharmaceutically acceptable salt thereof including an acid addition salt with hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, and naphthalenesulfonic acid.

In an embodiment of the present invention, 3-[(4-biphenylyloxy)methyl]piperidine may be a 3-[(4-biphenylyloxy)methyl]piperidine hydrochloride.

In an embodiment of the present invention, the composition for aiding an anti-cancer agent or radiation therapy may further include additives such as a pharmaceutically acceptable adjuvant, for example, a carrier, a stabilizer, a buffering agent, and an emulsifier, if desired.

The cancer to which the composition according to an embodiment of the present invention may be applied in combination with the anti-cancer agent or radiation therapy may include gastric cancer, lung cancer, prostate cancer, colon cancer, and breast cancer, but is not limited thereto. In particular, the composition according to an embodiment of the present invention may be effectively used in the treatment of a lung cancer in which the high Nrf2 activity is maintained due to mutation of gene for Keap1 capable of inhibiting the Nrf2.

According to a disclosure of the present invention, there is provided an Nrf2 inhibitor screening vector including an antioxidant response element (ARE) site having a nucleotide sequence of SEQ ID NO: 9 or SEQ ID NO: 10 and a nucleotide sequence coding for a reporter gene.

In an embodiment of the present disclosure, the reporter gene may be a gene coding for luciferase.

In an embodiment of the present disclosure, the vector may further include a selectable marker such as an antibiotic-resistance gene.

The ARE is a cis-acting regulatory element in the promoter region of several genes coding for proteins including antioxidant enzymes. The Nrf2 binds to the ARE sequence to induce transcriptional activation in downstream genes. Accordingly, a substance that binds to the ARE sequence and inhibits transcriptional activity of the Nrf2 may be suitable for an anti-cancer therapeutic adjuvant.

The vector according to an embodiment of the present disclosure includes an oligomer including the ARE site to which the Nrf2 or Nrf2 inhibitor strongly binds and a reporter gene that is regulated by a promoter sequence including the oligomer. Thus, a substance that binds to the ARE site to inhibit the binding of the Nrf2 to the ARE may be efficiently screened by measuring the expression level of the reporter gene.

According to another aspect of the present disclosure, there is provided a method of screening an Nrf2 inhibitor.

The method includes introducing an Nrf2 inhibitor screening vector having an ARE site of SEQ ID NO: 9 or SEQ ID NO: 10 and a nucleotide sequence coding for a reporter gene into an isolated cell, contacting the cell into which the vector is introduced with a candidate substance, and measuring an expression level of the reporter gene in the cell.

In an embodiment of the present disclosure, the isolated cell may be a cancer cell with high Nrf2 activity, wherein the cancer cell may be selected from the group consisting of gastric cancer cell, lung cancer cell, prostate cancer cell, colon cancer cell, breast cancer cell, and blood cancer cell.

In an embodiment of the present disclosure, the introduction of the screening vector into the isolated cell includes transformation or transfection, but is not limited thereto. Any method that is commonly used in the art may be used.

In an embodiment of the present disclosure, the reporter gene may be a gene coding for luciferase or fluorescent protein.

In an embodiment of the present disclosure, contacting the cell with the candidate substance may be performed in a medium in which the growth of the cell can be maintained.

In an embodiment of the present disclosure, the expression level of the reporter gene may be measured by measuring the amount of mRNA or protein expressed from the reporter gene.

The method of screening the Nrf2 inhibitor according to an embodiment of the present disclosure may further include comparing the expression level of the reporter gene measured in the cell contacted with the candidate substance with that measured in a control, and selecting the candidate substance as an Nrf2 inhibitor if the expression level measured in the cell contacted with the candidate substance is less than that measured in the control. The control is a cell treated under the same conditions except that the cell does not contact with the inhibitor candidate substance.

In an embodiment of the present disclosure, measuring an expression level of the reporter gene may be conducted using an antibody that specifically recognizes protein expressed from the reporter gene or fragments thereof, or a probe or primer that specifically recognizes nucleic acid coding for the protein expressed from the reporter gene or fragments thereof.

In an embodiment of the present disclosure, measuring an expression level of the reporter gene may be conducted using a method including, reverse transcription-polymerase chain reaction (RT-PCR), competitive RT-PCR, real time RT-PCR, northern blotting, western blotting, ELISA, radiation immunoassay, or immunoprecipitation.

In an embodiment of the present disclosure, if the reporter gene is a gene coding for luciferase or fluorescent protein, the expression level of the reporter gene may be measured by measuring direct emission or fluorescence.

### Advantageous Effects of Invention

The Nrf2 inhibitor may be efficiently screened using a method of screening an Nrf2 inhibitor according to an embodiment of the present disclosure. The composition comprising the Nrf2 inhibitor obtained by the screening method may be applied in combination with an anti-cancer agent or radiation therapy as an adjuvant thereto for synergistically increasing the effect of the anti-cancer agent therapy or radiation therapy.

### Brief Description of Drawings

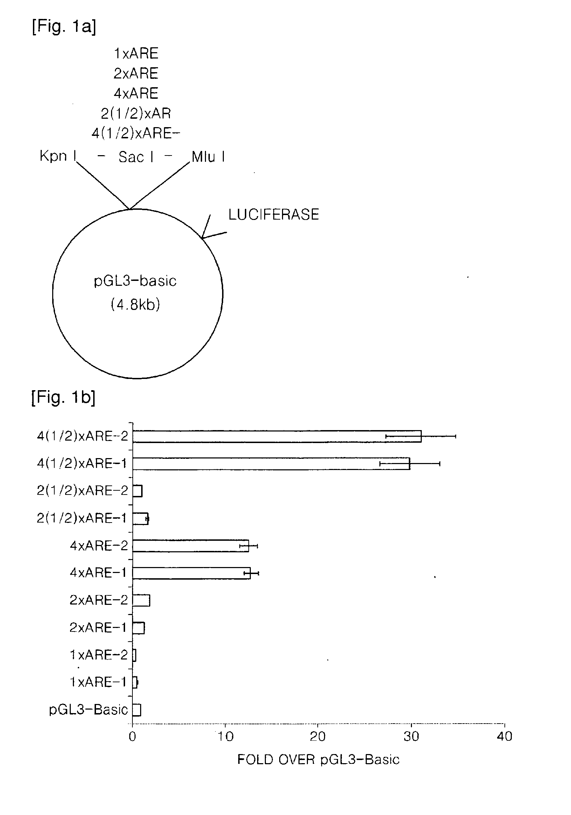

FIG. 1 a shows a schematic structure of a pGL3 luciferase plasmid, according to an embodiment of the present invention into which various types of oligomers including an antioxidant response element (ARE) site are inserted.
FIG. 1b shows luciferase activities measured in a cell transformed by the pGL3 luciferase plasmid shown in FIG. 1a. A pGL3 luciferase plasmid including 4(1/2)xARE that exhibits the highest luciferase activity was selected as a plasmid for screening an Nrf2 inhibitor.

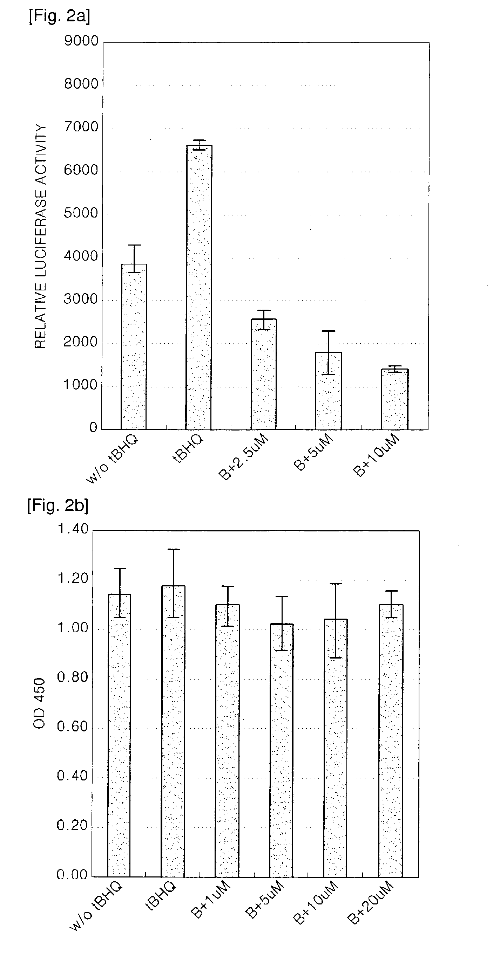

FIGS. 2a and 2b show that 3-[(4-biphenylyloxy)methyl]piperidine hydrochloride, an Nrf2 inhibitor according to an embodiment of the present invention, inhibits the activity of luciferase in a dose-dependent manner and does not have cell toxicity. In FIGS. 2a and 2b, B in the X-axis refers to tBHQ.

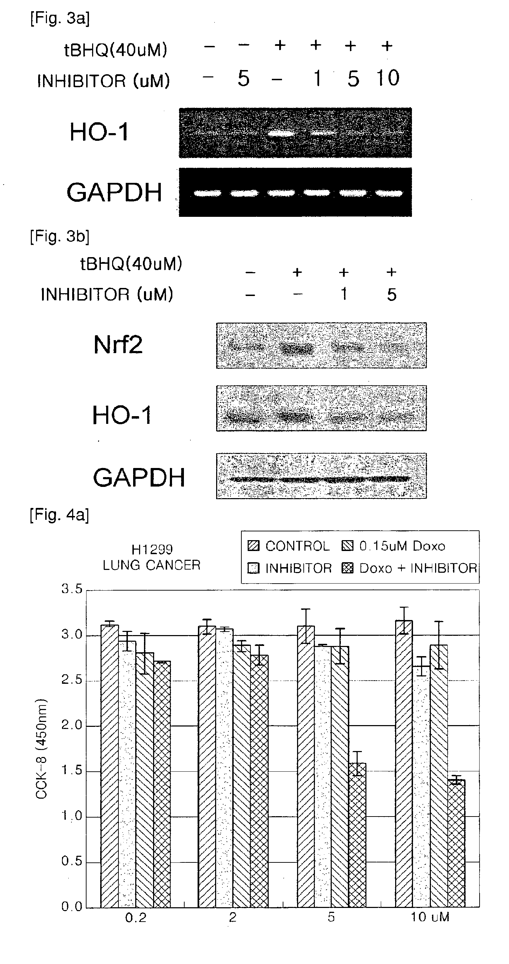

FIG. 3a shows that the expression of HO-1 mRNA is inhibited in a dose-dependent manner by inhibiting the Nrf2 transcription factor as the 3-[(4-biphenylyloxy)methyl]piperidine hydrochloride, an Nrf2 inhibitor according to an embodiment of the present invention, acts on the ARE site. In order to normalize the expression level, GAPDH primers were used as a control.
FIG. 3b shows the result of western blotting illustrating that the expression of Nrf2 protein and HO-1 protein is inhibited by inhibiting the Nrf2 transcription factor as 3-[(4-biphenylyloxy)methyl]piperidine hydrochloride, an Nrf2 inhibitor according to an embodiment of the present invention, acts on the ARE site. In order to normalize the expression level, a GAPDH antibody was used as a control.

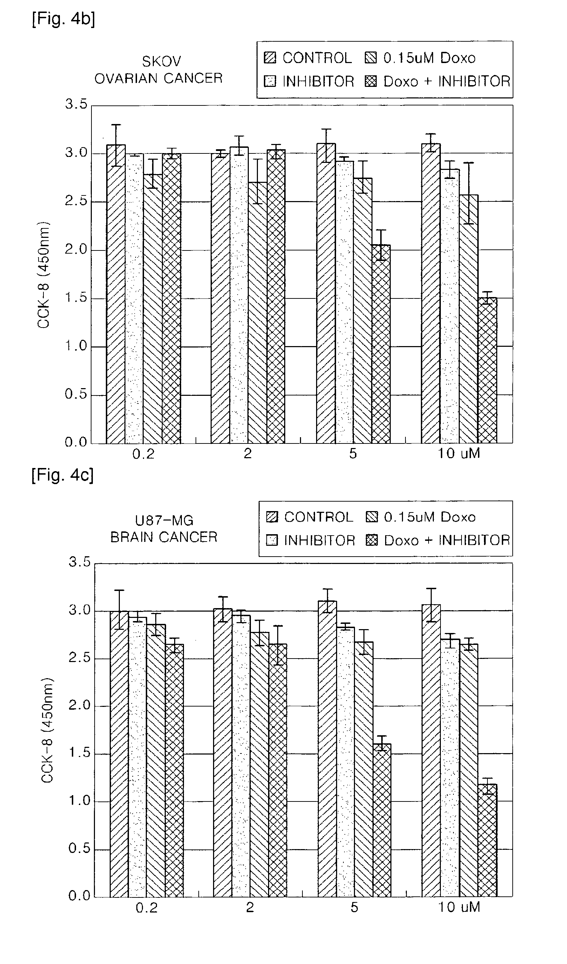

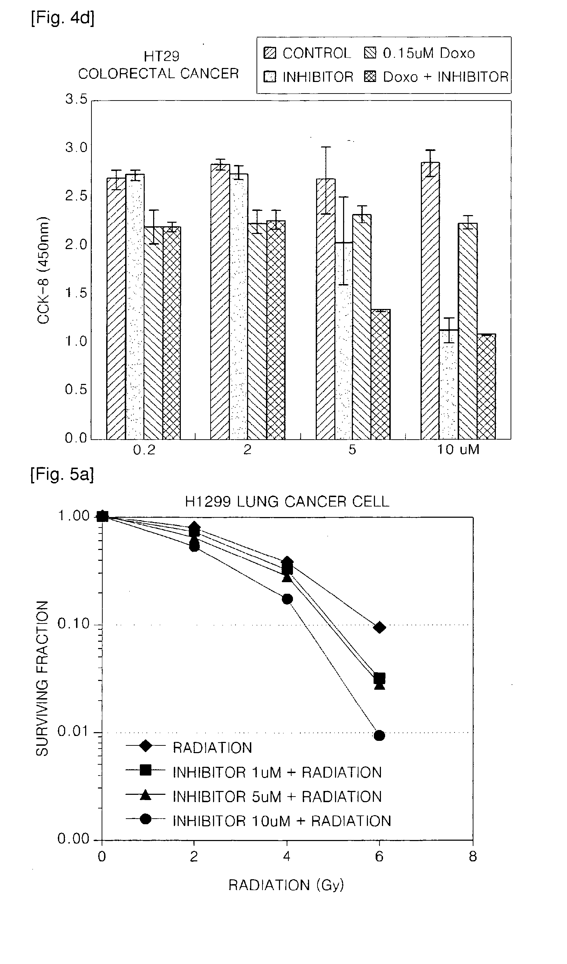

FIGS. 4a to 4d show that the apoptotic effect of an anti-cancer agent Doxorubicin on lung cancer, ovarian cancer, brain cancer, and colorectal cancer cells, increases in a dose-dependent manner by treating the cancer cells with 3-[(4-biphenylyloxy)methyl]piperidine hydrochloride, an Nrf2 inhibitor according to an embodiment of the present invention.

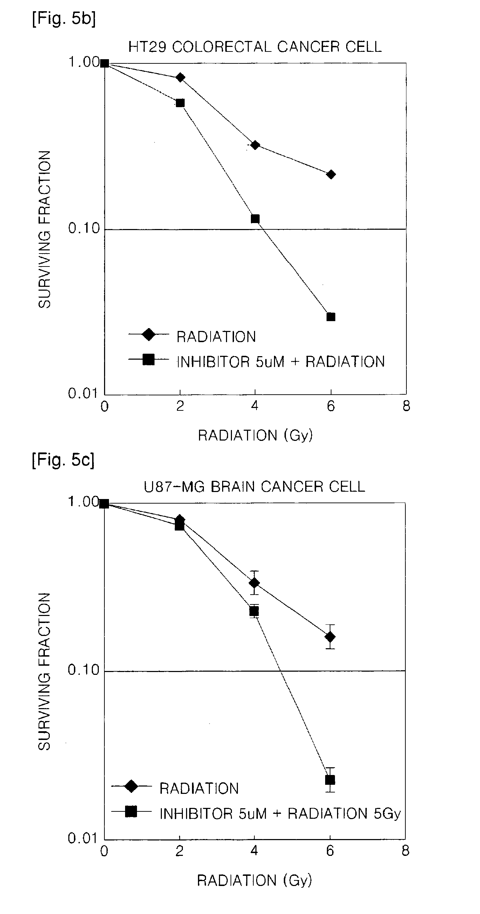

FIGS. 5a to 5c show increase in sensitivity of lung cancer, brain cancer and colorectal cancer cell lines to radiation measured using a Clonogenic method, when the cancer cell lines are treated with 1, 5, and 10 µM 3-[(4-biphenylyloxy)methyl]piperidine hydrochloride, an Nrf2 inhibitor according to an embodiment of the present invention, exposed to 2, 4, and 6Gy radiation, respectively, and stained with methylene blue.

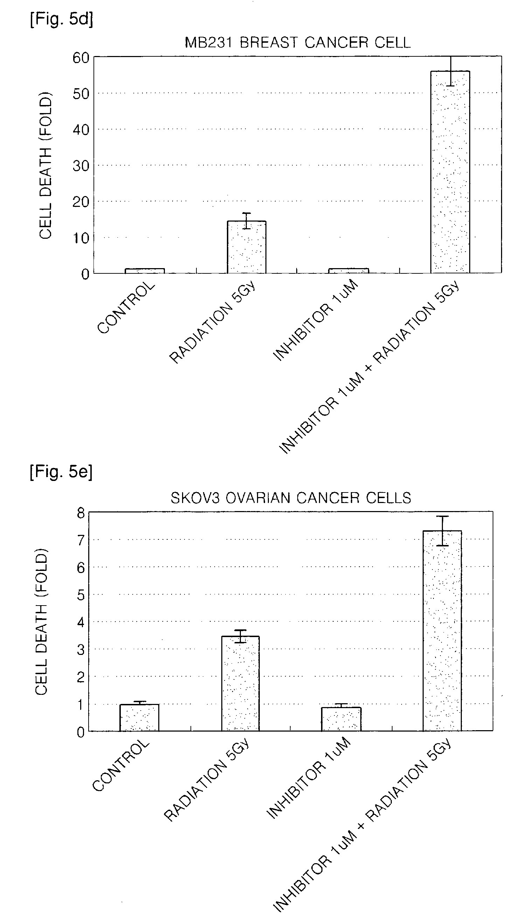

FIGS. 5d and 5e show increase in apoptotic effect on breast cancer cell lines and ovarian cancer cell lines measured using FACS, when the cancer cell lines are treated with 1 µM 3-[(4-biphenylyloxy)methyl]piperidine hydrochloride, an Nrf2 inhibitor according to an embodiment of the present invention, exposed to 5Gy radiation, and stained with propidium iodide.

### Mode for the Invention

Hereinafter, the present invention will be described in more detail.

### Comparative Example 1. Screening Nrf2 inhibitor

### (1) Preparation of Nrf2 inhibitor screening vector

Oligomers including an ARE site (SEQ ID NOS: 1 to 10, Bioneer Corp., Ltd., Korea) were synthesized and treated with Kpnl and M1 ul restriction enzymes. The oligomers were inserted into a pGL3 luciferase plasmid (Promega, USA) treated with the Kpnl and M1ul restriction enzymes using a ligase (Promega, USA) to prepare ARE-pGL3 luciferase. FIG. 1 a shows a schematic structure of the ARE-pGL3 luciferase plasmid. Besides the plasmid, pcDNA3.1 (Invitrogen, USA) plasmid including a Neo gene was used together as a selection plasmid to select a transformed cell using G418 (neomycin) as a selectable factor.

### (2) Cell culture, transformation, and luciferase assay

HEK293 cells were purchased from the American Type Culture Collection (ATCC) and cultured in a Dulbecco's modified Eagle's medium (DMEM) supplemented with 10% fetal bovine serum (FBS), 100 *µg*/ml of streptomycin, and 100 units/ml of penicillin under 5% CO₂ conditions at 37°C. A transformation by which the ARE-pGL3 luciferase was introduced into the HEK293cell was performed using Lipofectamine 2000™ (Invitrogen, Calsbad, CA, USA). Activity of luciferase was measured using a Luciferase Assay Kit (Promega, Medison, WI, USA). FIG. 1b shows activities of luciferase measured in cells transformed by a plasmid including one of 1xARE-1 to 4(1/2)xARE-2 oligomers having sequences of any one of SEQ ID NOS: 1 to 10. Based on the result, ARE-pGL3 luciferase plasmid including 4(1/2)xARE-2 having a sequence of SEQ ID NO: 10 was used as a plasmid for screening an Nrf2 inhibitor.

### (3) Screening of Nrf2 inhibitor

After the transformation performed according to Example 1-(2), transformed HEK293 cells that expressed both ARE-pGL3 luciferase and pcDNA3.1 were selected using a culture medium including G418 (600*µg*/ml) and used as a cell line for screening the Nrf2 inhibitor. 20,000 cells were inoculated into each well of a 96-well plate and cultured for 24 hours under the conditions described above in Example 1-(2). Then, it was found that the activity of the luciferase increased twice by adding to the culture tertiary butylhydroquinone (tBHQ) that activates the ARE. Transformed cells that stably expressed the ARE-pGL3 luciferase plasmid were selected, and 8,000 candidate substances, low molecular weight single compounds were used to screen the Nrf2 inhibitor. Each candidate substance was added to each well to a final concentration of 10 µM. The wells were incubated for 18 hours, and the luciferase activity was measured using a Victor 3 Luminometer (Perkin Elmer, USA). Based on the result, a low molecular weight compound, 3-[(4-biphenylyloxy)methyl]piperidine hydrochloride that inhibits the activity of the luciferase was selected.

As shown in FIG. 2a, the selected 3-[(4-biphenylyloxy)methyl]piperidine hydrochloride inhibited the activity of the luciferase in a concentration-dependent manner. In addition, in order to confirm that the inhibition of the luciferase activity is not caused by the death of a cell but by the Nrf2 inhibitor, absorbance was measured using a CCK-8 viability assay kit (Dojindo, Japan) and a spectrometer (Lab Systems, USA) at 450 nm. As shown in FIG. 2b, the selected 3-[(4-biphenylyloxy)methyl]piperidine hydrochloride does not have cell toxicity.

### Comparative Example 2. Identification of active site of selected Nrf2 inhibitor

In order to identify whether 3-[(4-biphenylyloxy)methyl]piperidine hydrochloride that was selected as the Nrf2 inhibitor in comparative Example 1 acted on the ARE site, a semi-quantitative RT-PCR and western blotting were conducted.

### (1) RNA extraction and semi-quantitative RT-PCR

After 2 hours from the treatment of 3-[(4-biphenylyloxy)methyl]piperidine chloride, the selected Nrf2 inhibitor, RNA expression was assayed in order to measure the increase in the amount of heme oxygenase (HO)-1 mRNA by tBHQ treatment. mRNA was isolated from the cells treated with 3-[(4-biphenylyloxy)methyl]piperidine hydrochloride, using an RNA extraction kit (Qiagen, USA) and identified using a spectrometer (Amersham Biosciences, USA). cDNA was obtained from the mRNA using a reverse transcriptase (Promega, USA), and PCR was conducted using the cDNA as a template and primers having sequences SEQ ID NOS: 11 and 12 to measure the expression level of HO-1 mRNA. The PCR was performed with 25 cycles, each of which consisting of a denaturation at 94°C for 30 seconds, an annealing at 55°C for 30 seconds, and an extension at 72°C for 1 minute. The DNA obtained using the PCR was electrophoresed in a 1.5% agarose gel, stained using ethidium bromide, and observed under UV rays. FIG. 3a shows expression levels of HO-1 from cells treated with the selected inhibitor and tBHQ. It is believed that the 3-[(4-biphenylyloxy)methyl]piperidine hydrochloride that is selected as the Nrf2 inhibitor using the ARE-pGL3 luciferase plasmid in Example 1 acted on the ARE site to inhibit the activity of the Nrf2 transcription factor.

### (2) Western blotting

In order to identify whether the 3-[(4-biphenylyloxy)methyl]piperidine hydrochloride, the selected Nrf2 inhibitor, acted on the ARE site to inhibit the binding of the Nrf2 transcription factor, and thereby inhibiting the expression of antioxidant enzymes, protein expression was analyzed. The increase in the amount of HO-1 protein by the tBHQ treatment was assayed after treating cells with the 3-[(4-biphenylyloxy)methyl]piperidine hydrochloride followed by treatment with tBHQ with a 2-hour gap between the treatments. Cytoplasmic fractions were obtained from the cells treated as described above using a method commonly used in the art. The fractions were separated using a 10% gradient SDS-PAGE, and then transferred to a nitrocellulose membrane (BioRad, Hercules, CA, USA). The membrane was blocked with a 5% skim milk and screened with an anti-Nrf2 antibody (Abcam, USA) and a HO-1 antibody (Oncogene Research Products, USA). The result was visualized by a reaction with a horseradish peroxidase-conjugated anti-rat IgG antibody (Santa Cruz Biotechnology, Inc.) using a thermostat and then with an ECL system (GE, USA). FIG. 3b shows that the amounts of the Nrf2 and HO-1 protein which were increased by the treatment with tBHQ were reduced by the treatment with the 3-[(4-biphenylyloxy)methyl]piperidine hydrochloride in a concentration-dependent manner.

### Example 1. Cancer cell-killing effect by a combination of Nfr2 inhibitor and anti-cancer agent

H1299 lung cancer cells, SKOV3 ovarian cancer cells, U87-MG brain cancer cells, and HT29 colorectal cancer cells were respectively inoculated into a 96-well plate including a Rosewell Park Memorial Institute medium 1640 (RPMI 1640) supplemented with 10% FBS, 100 *µg*/ml of streptomycin, and 100 unit/ml of penicillin. Each well was treated with 0.2, 2, 5, and 10 µM 3-[(4-biphenylyloxy)methyl]piperidine hydrochloride, and then treated with 0.15 µM Doxorubicin, an anti-cancer agent after 2 hours therefrom. The wells were cultured under 5% CO₂ conditions at 37°C for 48 hours, and the killing effect of the cancer cells was assayed using a CCK-8 viability assay kit (Dojindo, Japan). FIGS. 4a to 4d show that the effect of the 3-[(4-biphenylyloxy)methyl]piperidine hydrochloride on the viability of each of the cancer cell lines was negligible, but the killing effect of a combination of the 3-[(4-biphenylyloxy)methyl]piperidine hydrochloride and Doxorubicin on the cancer cells significantly increased in a concentration-dependent manner compared to that of a treatment with Doxorubicin alone.

### Example 2. Cancer cell-killing effect by a combination of Nfr2 inhibitor and radiation therapy

### (1) Lung cancer, brain cancer, and colorectal cancer cells

H1299 lung cancer cells, U87-MG brain cancer cells, or HT29 colorectal cancer cells were respectively inoculated into a 6 cm plate including a RPMI 1640 supplemented with 10% FBS, 100 *µg*/ml of streptomycin, and 100 unit/ml of penicillin. The plate was treated with the selected 1, 5, and 10 µM 3-[(4-biphenylyloxy)methyl]piperidine hydrochloride, the Nrf2 inhibitor, exposed to 2, 4, and 6 Gy -radiation, and cultured under 5% CO₂ conditions at 37°C for 7 days. The cultured cells were washed with a phosphate buffered saline (PBS) and stained with a 1% methylene blue in 100% methanol. Then, the number of alive cells that formed colonies was calculated three times. FIGS. 5a to 5c show that a combined application of the 3-[(4-biphenyloxy)methyl]piperidine chloride and radiation therapy increased or synergistically increased the sensitivity of cells to radiation.

### (2) Breast cancer and ovarian cancer cells

In order to measure whether a treatment of MB231 breast cancer cells and SKOV3 ovarian cancer cells with a combination of 3-[(4-biphenylyloxy)methyl]piperidine chloride, the selected Nrf2 inhibitor, and radiation therapy increase or synergistically increase the killing of cancer cells by apoptosis, the cancer cells were treated with 1 µM 3-[(4-biphenylyloxy)methyl]piperidine hydrochloride, exposed to 5 Gy radiation, and cultured under 5% CO₂ conditions at 37°C for 48 hours. The cultured cells were collected and stained with propidium iodide, and cell cycles were analyzed using a fluorescence activated cell sorting (FACS). FIGS. 5d and 5e show that the killing effect of the combination on the MB231 breast cancer cells increased by 4.3 times and the killing effect of the combination on the SKOV3 ovarian cancer cells increased by twice when compared to that of a 5Gy radiation therapy.
## Claims

1. A composition comprising 3-[(4-biphenylyloxy)methyl]piperidine or a pharmaceutically acceptable salt thereof for use in improving the therapeutic effect of an anti-cancer agent or radiation therapy by increasing sensitivity of cancer cells to the anti-cancer agent or radiation therapy when the composition is used in combination with the anti-cancer agent or radiation therapy.

2. The composition according to Claim 1, wherein the anti-cancer agent is selected from the group comprising cysplatin, doxorubicin, vinblastine, fluorouracil, irinotecan, paclitaxel, docetaxel, vinorelbine, and gemcitabine.

## Patentansprüche

1. Zusammensetzung, umfassend 3-[(4-Biphenylyloxy)methyl]-piperidin oder ein pharmazeutisch annehmbares Salz hiervon, zur Verwendung für die Verbesserung der therapeutischen Wirkung eines Antikrebsmittels oder von Strahlentherapie durch Erhöhung der Sensitivität von Krebszellen gegenüber dem Antikrebsmittel oder der Strahlentherapie, wenn die Zusammensetzung in Kombination mit dem Antikrebsmittel oder der Strahlentherapie eingesetzt wird.

2. Zusammensetzung nach Anspruch 1, wobei das Antikrebsmittel aus der Gruppe umfassend Cisplatin, Doxorubicin, Vinblastin, Fluorouracil, Irinotecan, Paclitaxel, Docetaxel, Vinorelbin und Gemcitabin ausgewählt ist.

## Revendications

1. Composition comprenant de la 3-[(4-biphénylyloxy)méthyl)pipéridine ou un sel pharmaceutiquement acceptable de celle-ci pour utilisation dans l'amélioration de l'effet thérapeutique d'un agent anticancéreux ou d'une radiothérapie par augmentation de la sensibilité des cellules cancéreuses à l'agent anticancéreux ou à la radiothérapie lorsque la composition est utilisée en combinaison avec l'agent anticancéreux ou la radiothérapie.

2. Composition selon la revendication 1, dans laquelle l'agent anticancéreux est choisi dans le groupe comprenant le cisplatine, la doxorubicine, la vinblastine, le fluorouracile, l'irinotécan, le paclitaxel, le docétaxel, la vinorelbine et la gemcitabine.
